# EUROPEAN PATENT APPLICATION

(11) **EP 4 361 639 A1**
(43) Date of publication of application: **01.05.2024**
(21) Application number: 22827965.9
(22) Date of filing: 14.03.2022
(51) Int. Cl.: G01N 35/00

(54) **AUTOMATED ANALYSIS DEVICE**

(30) Priority: 24.06.2021 JP 2021104580
(71) Applicant: HITACHI HIGH-TECH CORPORATION, Tokyo 105-6409 (JP)
(72) Inventor: YAMAMOTO, Takashi, Tokyo 105-6409 (JP)
(74) Representative: MERH-IP Matias Erny Reichl Hoffmann Patentanwälte PartG mbB
(86) International application number: PCT/JP2022/011439
(87) International publication number: WO 2022/270044

(57) **Abstract**

[Task] To provide a technique that allows efficient processing of multiple-type analysis.

[Solution] The automatic analyzer includes a mechanism for analyzing a specimen with respect to each of multiple analysis types, a processor which receives specimen analysis request information based on an input by a user, and controls the mechanism to execute an analytical operation in accordance with a designated analysis type for processing the corresponding analysis, and a storage device which stores evaluation setting information for prioritizing the analysis request information that involves an order of the analysis. The evaluation setting information (score table 50) has an evaluation value (score) that reflects priority in the analysis order set on each of multiple information elements relating to the analysis. The multiple information elements include at least a specimen type and the analysis type. The processor calculates the evaluation value set on each of the received multiple analysis request information based on the evaluation setting information, and determines an order of priority for assignment of the analysis processing in accordance with the evaluation value.

## Description

### Technical Field

The present invention relates to an automatic analyzer.

### Background Art

In the past, an automatic analyzer with a dedicated configuration has been separately used for each test classification item (in other words, analysis type) such as biochemistry, immunity, electrolyte, and coagulation. However, in recent years, a type of automatic analyzer has been developed, which is mounted with mechanisms that allows the automatic analyzer to deal with multiple types of measurements, analyses, inspections, and the like (these are sometimes collectively referred to as analysis) alone. For example, some automatic analyzer is mounted with a module for biochemical analysis, a module for immunity analysis, a common module, and the like. The common module is a mechanism that can be used commonly regardless of analysis types.

Japanese Unexamined Patent Application Publication No. 2014-9972 (patent literature 1) describes an apparatus that measures multiple test classification items within the apparatus alone, and that has a setting means for setting priority order of samples to be measured in test classification units of biochemistry, immunity, electrolyte, and coagulation, and has a control unit that determines order of measurement of the test items of the samples based on priority order of the test classification units and based on priority order of measurement categories (emergent, urgent, and general) of the sample.

### Citation List

### Patent Literature

PTL 1: Japanese Unexamined Patent Application Publication No. 2014-9972.

### Summary of Invention

### Technical Problem

In order to provide a function of multiple-type analysis, such as biochemistry and immunity, in one automatic analyzer, and to achieve size reduction, it is necessary to share a mechanism used for the function for each analysis type without specializing the mechanism as much as possible. However, in the case of the automatic analyzer having such a shared mechanism configuration, when analysis of each analysis type for multiple analysis requests is performed, competition may occur between the mechanisms used. If such competition occurs, test time may be prolonged, and a sample and a reagent may degrade over time.

For such an automatic analyzer, in order to efficiently deal with multiple analysis requests, various pieces of information and various viewpoints, such as a sample type and sample residual amount, are involved in analysis processing, in addition to the analysis type. In the past, in assignment of multiple analysis requests to analysis processing (multiple corresponding mechanisms), there is room for improvement to achieve more efficient assignment in comprehensive consideration of multiple pieces of information and multiple viewpoints.

An object of the invention is to provide a technique that allows an automatic analyzer, which has a multiple-type analysis function implemented in the automatic analyzer alone, to process multiple-type analysis efficiently.

### Solution to Problem

A typical embodiment of the present invention has the following configuration. An automatic analyzer of the embodiment comprises a mechanism for analyzing a sample with respect to each of multiple analysis types, a processor that receives sample analysis request information based on a user input, and controls the mechanism to execute an analytical operation in accordance with a specified analysis type based on the analysis request information to process the corresponding analysis, and a storage device that stores evaluation setting information for prioritizing the analysis request information involved in order of the analysis, where the evaluation setting information has an evaluation value that reflects priority in the analysis order set on each of values of multiple information elements relating to the analysis, the multiple information elements include at least a sample type and the analysis type, and the processor calculates an evaluation value on each of multiple pieces of the received analysis request information based on the evaluation setting information, and determines order of priority for assignment of the analysis processing in accordance with the evaluation value. Advantageous Effects of Invention

According to the typical embodiment of the invention, an automatic analyzer, which has a multiple-type analysis function implemented in the automatic analyzer alone, can efficiently process multiple-type analysis. Other problems, configurations, effects, and the like will be presented in the description of embodiment.

### Brief Description of Drawings

[Fig. 1] Fig. 1 schematically illustrates a structure of an automatic analyzer.
[Fig. 2] Fig. 2 is an exemplary functional block structure of the automatic analyzer.
[Fig. 3] Fig. 3 represents an example of multiple information elements to be used for prioritization that involves analytical processing.
[Fig. 4] Fig. 4 represents an exemplary structure of a score table to be used for prioritization that involves the analytical processing.
[Fig. 5] Fig. 5 represents another exemplary structure of the score table.
[Fig. 6] Fig. 6 represents another exemplary structure of the score table.
[Fig. 7] Fig. 7 represents a main processing flow.
[Fig. 8] Fig. 8 represents an example of analysis request information.
[Fig. 9] Fig. 9 represents an example of a score of the analysis request information.
[Fig. 10] Fig. 10 represents an example of assignment in the order of input according to a comparative example of the embodiment.
[Fig. 11] Fig. 11 represents an assignment in the order of priority.
[Fig. 12] Fig. 12 represents an exemplary screen of a score table setting function according to a modification 1.
[Fig. 13] Fig. 13 represents an exemplary screen of a score table setting function according to a modification 2.

### Description of Embodiments

Hereinafter, one embodiment of the invention will be described in detail with reference to the drawings. In the drawings, the same parts are designated by the same sign in principle, and duplicated description is omitted. In the drawings, each component may not be shown in its actual position, size, shape, range, or the like in order to easily understand the invention. When processing with a program is described, the program, a function, a processing section, etc. may be described as a main body for illustrative purposes, and the main body in a form of hardware includes a processor, or a controller, device, computer or system including the processor. The computer executes processing by the processor according to a program read out on a memory while using resources such as the memory and a communication interface as appropriate. This enables a predetermined function, processing section, and the like. The processor includes, for example, a semiconductor device such as CPU or GPU. The processor includes a device or a circuit that can perform predetermined operation. The processing is not limited to software program processing, but may also be implemented using a dedicated circuit. The usable dedicated circuit includes FPGA, ASIC, and CPLD. The program may be installed as data in the target computer in advance, or may be distributed as data from a program source to the target computer and installed therein. The program source may be a program distribution server on a communication network or a non-transitory computer-readable storage medium (e. g. , memory card) . The program may be configured of multiple modules. The computer system may not limitedly include one apparatus, but may include multiple apparatuses. The computer system may include a client server system, a cloud computing system, an IoT system, or the like. Various types of data and information are expressed and implemented, for example, by a table or a list, which however is not limitative. Expressions such as identification information, identifier, ID, name, and number can be replaced with each other.

### [Problems]

Some automatic analyzer of a related-art example has a function of prioritizing analysis by combining multiple factors, such as sample categories and test classification items, together as in an example of the patent literature 1. The sample category (in other words, sample type) includes, for example, urgent and general. The test classification items refer to analysis types such as biochemistry, immunity, and electrolyte. When such an automatic analyzer receives multiple analysis requests for multiple samples, it determines in which order the analysis processing is performed for the multiple analysis requests based on the concept of priority or priority order. The automatic analyzer determines the order, in which the multiple analysis requests are assigned to the analysis processing, considering factors such as a sample type and an analysis type. Such a concept may be referred to as prioritization below.

In the related-art example, a decision tree is used as a general technical means/method for the prioritization. The decision tree is an analysis method using a tree structure (in other words, a tree diagram) . For example, the decision tree has a structure, in which an element A branches into, for example, two elements B1 and B2, and the element B1 branches into, for example, two elements C1 and C2. In a more detailed example, the element A is a sample type, which branches into general (element B1) and urgent (element B2).

For the automatic analyzer, information elements, which must be considered for analysis processing, are being more diverse and complex. This makes it difficult to perform prioritization using the above decision tree method especially for the type of the automatic analyzer with multiple-type analysis functions implemented in the automatic analyzer alone. Furthermore, even if prioritization is performed using such a method, efficient assignment (in other words, analysis reservation) cannot necessarily be performed.

Therefore, the automatic analyzer of the embodiment, which is configured to enable multiple-type analysis in the automatic analyzer alone, is involved in prioritizing multiple analysis requests, and achieves efficient assignment while incorporating multiple information elements that increasingly become diverse and complex, resulting in reduction in testing time and suppression of degradation of a sample and a reagent over time.

The automatic analyzer of the embodiment uses a means/method using scores (in other words, evaluation values) described later as a technical means/method for achieving complex prioritization based on multiple factors. Consequently, the embodiment solves the problem that arises in case of using the decision tree. In other words, the embodiment facilitates and streamlines prioritization.

### Embodiment

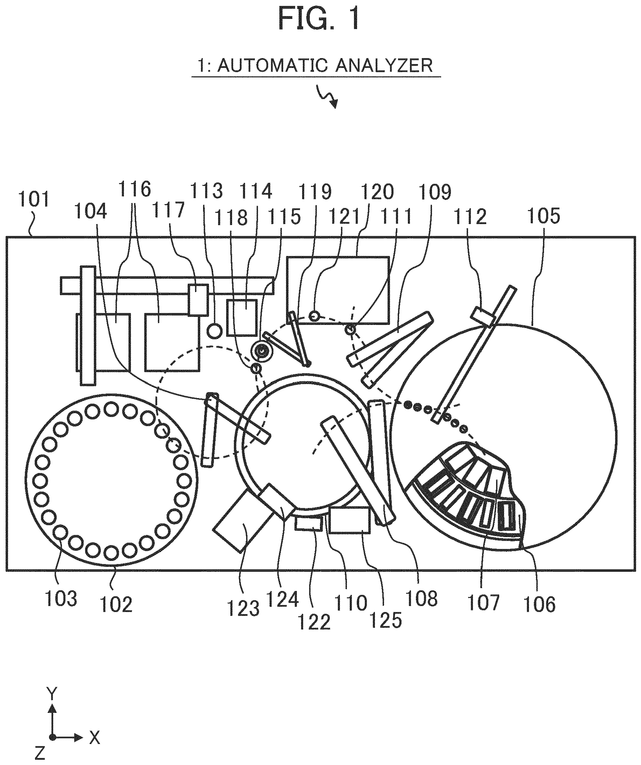

The automatic analyzer of the embodiment is now described using Figs1 to 11. The automatic analyzer of the embodiment is a type having a mechanism capable of performing multiple-type analysis. The automatic analyzer of the embodiment prioritizes multiple pieces of analysis request information for multiple samples with respect to order of analysis processing, and assigns (in other words, reserves) the information to analysis processing. At that time, the automatic analyzer of the embodiment calculates a score (in other words, evaluation value) for each piece of analysis request information based on a predetermined score table that is defined by taking into account multiple types of predetermined information elements related to the analysis processing, and performs prioritization based on that score. The score is an evaluation value for determining the prioritization, which is calculated by considering various information elements (in particular, priority/priority order of each information element).

In this score table (in other words, prioritization evaluation setting information), a score or a score calculation method is defined for an item and a value of each of multiple information elements, taking into consideration priority/priority order. For example, one of the information elements is an analysis type, and the priority depends on the analysis type. Scores are set according to the priority, taking into account that the cycle time or the like of the mechanism used depends on the analysis type. For example, one of the information elements is the sample residual amount or reagent residual amount, and the priority depends on the residual amount. The score is set according to the priority, taking into account the sample residual amount and the reagent residual amount. Priority between information elements is also taken into account in setting the score. For example, the priority goes from the highest to the lowest in order of sample type, analysis type, sample residual amount, and reagent residual amount. These will streamline the analysis comprehensively and totally.

### [Automatic Analyzer]

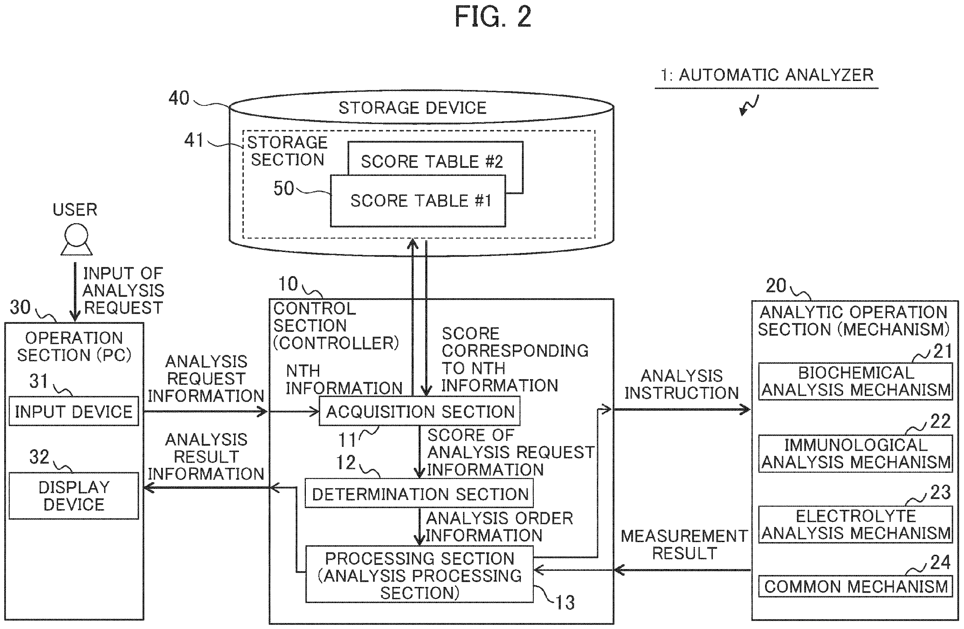

Fig. 1 shows a schematic configuration of an automatic analyzer 1 of the embodiment. Fig. 1 illustrates a configuration as seen from a top of a housing 101 of the automatic analyzer 1, showing the main components. Sections such as a control section, an operation section, and a storage section, which are mounted within or outside the housing 101, will be described with reference to Fig. 2 and other drawings mentioned later. A user (e.g., an operator such as a laboratory technician) operates and uses the automatic analyzer 1 to perform analysis and other operations.

The automatic analyzer 1 includes, on the top of the housing 101, a sample disc 102, a sample dispensing mechanism 104, a reagent storage 105, a reagent dispensing mechanism 108 for biochemical analysis, a reagent dispensing mechanism 109 for immunity analysis, a reaction disc (incubator) 110, a first reaction container transport unit 117, a reaction container disposal hole 113, an incubator block 114, a reaction liquid stirring unit 115, a reaction container tray 116, a second reaction container transport unit 119, an immunodetection unit 120, a stirring unit 122, a biochemical detection unit 123, a reaction cell cleaning mechanism 124, an electrolyte analysis unit 125, and the like.

The sample disc 102 is a sample container holding mechanism, in which multiple sample containers 103 can be installed in a ring. The sample container 103 stores samples. In sample dispensing, the sample disc 102 rotates to transport a target sample container 103 to an access position of the sample dispensing mechanism 104 (in other words, a dispensing position, a sample aspiration position).

The sample dispensing mechanism 104 includes a rotary drive mechanism, a vertical drive mechanism, and a dispensing probe. The sample dispensing mechanism 104 moves between a sample aspiration position on the sample disc 102 and a sample discharge position on the reaction disc 110 using the rotary drive mechanism and the vertical drive mechanism.

The reagent storage 105 includes a reagent disk 106 and a reagent bottle holding section 107. In general, the reagent storage has a cold storage function to enhance on-board stability of reagents. Reagent holding sections 107 are arranged in a double ring on the reagent disc 106 so that multiple reagent bottles can be held. The reagent bottle holds a container containing a reagent. The reagent disc 106 has a rotational drive mechanism and moves each reagent bottle to a predetermined position on the circumference through rotary motion.

The automatic analyzer 1 of the embodiment separately has the reagent dispensing mechanism 108 for biochemical analysis and the reagent dispensing mechanism 109 for immunity analysis. These mechanisms are exemplary mechanisms for each type of analysis. Each of the reagent dispensing mechanisms (the reagent dispensing mechanism 108 and the reagent dispensing mechanism 109) includes a rotary drive mechanism, a vertical drive mechanism, and a dispensing probe. Each reagent dispensing mechanism rotates to a position of a predetermined type of reagent bottle on the reagent disc 106, and lowers to aspirate a predetermined amount of the reagent. After aspirating the reagent, the reagent dispensing mechanism rises and then rotates to a reagent discharge destination and lowers to discharge the reagent. For example, the reagent discharge destination is a predetermined reaction container on the reaction disc 110 for biochemical analysis, which is placed at a reagent discharge position 111 for immunity analysis.

An arm 112 (also referred to as a stirrer), which is a magnetic particle stirring arm serving as a stirring means, is set on the reagent disc 106. The arm 112 moves to an upper region of a reagent bottle containing a magnetic particle solution to be stirred, lowers a magnetic particle stirring element of the arm 112, and rotates the magnetic particle stirring element to stir the magnetic particle solution. To prevent spontaneous precipitation of magnetic particles in the magnetic particle solution, the arm 112 performs the stirring immediately before reagent dispensing.

### [Analysis Type]

The automatic analyzer 1 of the embodiment includes multiple types of analysis (in other words, includes test classification items), i.e., three types of analysis including immunity analysis, biochemical analysis, and electrolyte analysis. Each analysis type is described below.

### [Immunity Analysis]

An exemplary flow of immunity analysis performed by the automatic analyzer 1 is as follows. Description is now given in order of processes (corresponding steps and operations) of the immunity analysis. The order of the processes of the immunity analysis is in summary as follows: Sample dispensing, reagent dispensing, reaction, and detection.

A first reaction container transport unit 117 has a drive mechanism in three axial directions of X, Y, and Z. The X axis and the Y axis are each defined to be in a horizontal direction, and the Z-axis is in a vertical direction. The first reaction container transport unit 117 moves above the reaction container disposal hole 113, the incubator block 114, the reaction liquid stirring unit 115, and the reaction container tray 116. The first reaction container transport unit 117 moves a reaction container from the reaction container tray 116 to a sample discharge position 118. The sample dispensing mechanism 104 dispenses a predetermined volume of a sample into the reaction container placed at the sample discharge position 118. Subsequently, the reaction container, into which that sample has been discharged, is moved to the reagent discharge position 111 by a second reaction container transport unit 119.

The second reaction container transport unit 119 includes a rotational drive mechanism, a vertical drive mechanism, and a reaction container gripping mechanism. The second reaction container transport unit 119 has a function of moving the reaction container to each reaction container placement position provided on a rotating orbit. The reaction container placement position includes a position of the reaction liquid stirring unit 115 and the sample discharge position 118.

The reagent dispensing mechanism 109 for immunity analysis dispenses a predetermined amount of reagent into the reaction container placed at the reagent discharge position 111. Subsequently, the reaction container is moved by the second reaction container transport unit 119 to a position of the reaction liquid stirring unit 115. After stirring of the reaction liquid at the position of the reaction liquid stirring unit 115, the reaction container is moved to the incubator block 114 by the first reaction container transport unit 117. The incubator block 114 is temperature-controlled to an appropriate temperature to promote the reaction of the sample and the reagent. When the reaction process between the sample and the reagent in the reaction container on the incubator block 114 is completed, the reaction container is moved to the sample discharge position 118 by the first reaction container transport unit 117.

Subsequently, the reaction container is moved by the second reaction container transport unit 119 to the reaction liquid aspiration position 121, which is located closer to the second reaction container transport unit 119 (on the lower side along the Y axis in the drawing) in the immunodetection unit 120. Subsequently, the reaction liquid in the reaction container is aspirated into a detection section (in other words, optical measurement mechanism) in the immunodetection unit 120, and a reaction signal is measured by the detection section. After measurement of the reaction signal, the reaction container is moved to the sample discharge position 118 by the second reaction container transport unit 119, and then discarded by the first reaction container transport unit 117 into the reaction container disposal hole 113.

### [Biochemical Analysis]

An exemplary flow of biochemical analysis performed by the automatic analyzer 1 is as follows. The order of the processes of the biochemical analysis is in summary as follows: Sample dispensing, reagent dispensing, reaction, and detection.

First, the sample dispensing mechanism 104 dispenses a predetermined amount of a sample into a predetermined reaction container on the reaction disc 110. Subsequently, the reaction disc 110 rotates to move the reaction container, into which the sample has been discharged, to an access position of the reagent dispensing mechanism 108. The reagent dispensing mechanism 108 dispenses a predetermined amount of a reagent into the reaction container, into which the sample has been discharged. Subsequently, the reaction disc 110 rotates to move the reaction container, into which the sample and the reagent have been discharged, to a placement position of the stirring unit 122. Subsequently, the sample and the reagent in the reaction container are stirred by the stirring unit 122.

As with the incubator block 113, the reaction disc 110 is temperature-controlled to an appropriate temperature to promote the reaction of the sample and the reagent. When the reaction process between the sample and the reagent on the reaction disc 110 is completed, the reaction disc 110 rotates to move the reaction container containing the reaction liquid after completion of the reaction to a placement position of the biochemical detection unit 123. Subsequently, a reaction signal is measured by a detection section (in other words, an optical measurement mechanism) within the biochemical detection unit 123. After measurement of the reaction signal, the reaction liquid is discharged from the reaction container by the reaction container cleaning mechanism 124.

### [Electrolyte Analysis]

An exemplary flow of electrolyte analysis performed by the automatic analyzer 1 is as follows. The order of the processes of the electrolyte analysis is in summary as follows: Sample dispensing and detection.

First, the sample dispensing mechanism 104 dispenses a predetermined amount of a sample into a predetermined reaction container on the reaction disc 110. Subsequently, the reaction disc 110 rotates to move the reaction container, into which the sample has been discharged, to an access position of the electrolyte analysis unit 125. A detection section (in other words, an optical measurement mechanism) in the electrolyte analysis unit 125 then performs measurement. After the measurement, the sample is discharged from the electrolyte analysis unit 125.

The automatic analyzer 1 of the embodiment includes various mechanisms as described in the above example (including the components in Fig. 1) . The mechanisms each refer to an object that includes a physical component, and hardware, a circuit, etc. for drive or the like. The mechanism may partially include a software program.

### [Functional Block]

Fig. 2 shows an exemplary functional block structure of the automatic analyzer 1 of Fig. 1. Fig. 2 mainly shows components involved in control and processing. Fig. 2 collectively shows the above mechanisms as an analytic operation section 20, which performs operations involved in analysis. The processing involved in analysis (information processing, signal processing, arithmetic processing, etc.) is shown as processing performed mainly by a processing section 13 of a control section 10.

In the analytic operation section 20 of the automatic analyzer 1, the mechanisms are classified into mechanisms for each type of analysis, i.e., biochemical analysis mechanism 21, an immunological analysis mechanism 22, and an electrolyte analysis mechanism 23; and a common mechanism 24 that is sharable regardless of the analysis type. The biochemical analysis mechanism 31 is used only for biochemical analysis. Examples corresponding to the biochemical analysis mechanism 21 include the reagent dispensing mechanism 108 for biochemical analysis and the immunodetection unit 120 as described above. Examples corresponding to the immunological analysis mechanism 22 include the above reagent dispensing mechanism 109 for immunity analysis. Examples of the electrolyte analysis mechanism 23 include the above electrolyte analysis unit 125. The common mechanism 24 is a mechanism appropriately used in common for biochemical analysis, immunological analysis, and electrolyte analysis. Examples corresponding to the common mechanism 24 include the sample disc 102, the sample dispensing mechanism 104, and the reagent storage 105 as described above.

The automatic analyzer 1 includes the control section 10 and an operation section 30 in addition to the analytic operation section 30. A storage device 40 is connected to or built in the control section 10. The storage device 40 is, for example, a nonvolatile storage device. The storage device 40 may include, for example, a hard disk, a memory card, or a database server on a communication network. The control section 10, the analytic operation section 20, the operation section 30, and the storage device 40 are connected to each other by a predetermined communication interface and a signal line, and can perform input/output and communication of a signal or data with each other. The sections etc. may be connected to each other via a communication network such as a LAN.

The control section 10 controls operation of the whole automatic analyzer 1. The control section 10 controls analysis of each analysis type. In other words, the control section 10 is a controller. The control section 10 is implemented by, or configured of, a hardware board and a computer system, for example. The control section 10 includes an undepicted processor, memory, communication interface, and bus, for example. The processor includes, for example, CPU, ROM, or RAM. The processor executes predetermined processing according to a computer program read into the memory. This enables functions of an acquisition section 11 and other sections.

The control section 10 may be configured primarily in a form of hardware by a dedicated circuit board or primarily in a form of software by a computer system. When configured mainly by hardware, the control section 10 is enabled by integrating multiple arithmetic units, each executing processing, on a wiring board or in a semiconductor chip or package. When configured mainly by software, the control section 10 is enabled by executing a program to perform desired arithmetic processing on a computer with a high-speed general-purpose CPU and the like.

When a program configuring the control section 10, i.e., a computer program that causes the processor of the control section 10 to execute processing, is provided, the program is stored in a predetermined site. For example, the program may be stored in the storage device 40, in a memory within the control section 10, or in another external recording medium. The program may be delivered from a server or other device on a communication network and installed within the automatic analyzer 1. Further, the program may be, for example, updated as appropriate to allow a configuration of the existing automatic analyzer 1 (especially the control section 10) to be upgraded.

Various devices, circuits, and computers may be connected via wired or wireless communication, and data and information may be transmitted and received as appropriate. The control section 10 and the operation section 30 may each be configured as a computer system using multiple computers. For example, the acquisition section 11, the determination section 12, and the processing section 13 may be configured as separate computer systems and linked to each other through communication.

The operation section 30 is a section operated by a user. The operation section 30 can be implemented by, or configured of, a PC, for example. The operation section 10 is provided with, or externally connected to, an input device 31, a display device 32, and the like. The input device 31 is a mouse or a keyboard, for example. The display device 32 is a liquid crystal display, for example. The operation section 10 includes a processor, a memory, and a communication interface (not shown), for example. The operation section 30 and the control section 10 may be integrated in one computer system. The operation section 30 may be configured as a client computer, and the control section 10 as a server computer. The user can operate the input device 31 while viewing information on a screen including a graphical user interface (GUI) displayed on the display device 32 of the operation section 10 to input an instruction, a setting, etc. to the system of the automatic analyzer 1.

The storage device 40 has the storage section 41 including a memory area. The control section 10 controls the storage section 41. A score table 50 described later is beforehand set and stored in the storage section 41. The control section 10 appropriately reads and writes the score table 50 of the storage section 41 by the acquisition section 11. Although one or more score tables 50 can be set, the embodiment is described assuming only one score table 50 is used.

The storage device 40 or the control section 10 also stores setting information such as a temperature range corresponding to each mechanism of the analytic operation section 20, for example.

More specifically, the control section 11 includes the acquisition section 11, the determination section 12, and the processing section 13. The acquisition section 11 receives multiple pieces of analysis request information for multiple samples, and acquires a score for each analysis request from the score table 50 of the storage section 41 based on the relevant analysis request information. The determination section 12 prioritizes multiple pieces of analysis request information based on the acquired scores and assigns (in other words, reserves) the information to analysis processing (corresponding mechanism, cycle time, etc.) at the processing section 13, and thus determines the processing order of each analysis request. The processing section 13 performs the analysis processing for the multiple analysis requests according to the processing order determined as the analysis reservation. The processing section 13 gives analysis instructions to the analytic operation section 20. The mechanism of the analytic operation section 20 performs operations such as measurement according to the analysis instructions and returns measurement results, for example. The processing section 13 creates analysis result information of the analysis processing based on the measurement results, etc., and gives it to the operation section 30.

### [Processing and Operation of Each Section]

Processing and operations between the control section 10, the operation section 30, the storage device 40, and the analytic operation section 20 in Fig. 2 are now described. The arrow lines in the drawing represent, but not limited to, examples of connections and data/information transmission between the sections etc.

The user operates the input device 31 of the operation section 30. The operation section 30 requests information from the control section 10. The control section 10 transmits information of a screen or the like to the operation section 30. The operation section 10 displays a picture including a GUI on a display screen of the display device 32 based on the information from the control section 10. The user enters analysis request information on the screen of the display device 32. The screen has predetermined items for requesting analysis, and the user selects and enters information for each item, for example. The information that the user enters as analysis request information includes information to specify a sample to be analyzed, information to specify an item of analysis, information to specify a dilution factor, and the like. The operation section 30 sends analysis request information, prepared based on the information entered on the screen, to the control section 10.

In the embodiment, the automatic analyzer has a capability of considering the case where multiple pieces of analysis request information for multiple samples are generated in the same period of time (in other words, roughly at the same timing), and responding to such a case. In a specific example, the user may input multiple pieces of analysis request information for multiple samples on the screen. In this case, the multiple pieces of analysis request information are initially simply arranged in order of input. The control section 10 determines a processing order suitable for such pieces of analysis request information by prioritization based on the score table 50 and controls the information.

The control section 10 enters the multiple pieces of analysis request information received from the operation section 30 into a memory queue or the like in order of input (in other words, the order of reception) and holds the information.

The storage device 40 stores at least one score table 50 in the storage section 41. In the embodiment, the score table 50 is one score table 50 that has been initialized in advance by the business operator of the automatic analyzer 1. The score table 50 has fixed information content.

The acquisition section 11 of the control section 10 acquires a score of the analysis request information from the score table 50 of the storage section 41 in the storage device 40 based on the analysis request information notified from the operation section 30. At this time, the acquisition section 11 transmits information (illustrated as the Nth information) corresponding to an information element of the score table 50 in the analysis request information to the storage section 41, and acquires a score corresponding to the Nth information from the score table 50 of the storage section 41. Similarly, the acquisition section 11 acquires all the pieces of Nth information (N = 1 to M) for each piece of analysis request information. The acquisition section 11 may extract information included as attributes from the analysis request information, and may use that information as the Nth information to perform a search process with respect to the score table 50 of the storage section 41.

The acquisition section 11 totalizes the scores by adding the score for each Nth information (corresponding information element) for each piece of analysis request information. This provides a score for each piece of analysis request information. The acquisition section 11 notifies the determination section 12 of the score of the analysis request information (score for each piece of analysis request information).

The determination section 12 acquires a score for each piece of analysis request information for the multiple pieces of analysis request information notified from the acquisition section 11. The determination section 12 prioritizes; the multiple pieces of analysis request information using the scores. Specifically, the determination section 12 prioritizes the multiple pieces of analysis request information in descending order of the scores. The determination section 12 assigns the multiple pieces of prioritized analysis request information to analysis processing (corresponding mechanisms, cycles, etc.) of the processing section 13. In other words, the determination section 12 makes analysis reservations for the multiple pieces of analysis request information. As a result, the determination section 12 determines the processing order of the multiple pieces of analysis request information.

In a possible modification, the determination section 12 performs the processing up to determination of the priority order of the analysis request information, and the processing section 13 performs actual assignment of the analysis processing.

The control section 10, especially the processing section 13, appropriately sends an analysis instruction to each mechanism necessary for analysis operation of the analytic operation section 20. Each mechanism performs an operation such as measurement based on drive or the like according to the analysis instruction, and returns information such as measurement results. In particular, the measurement corresponds to operation of the immunodetection unit 120, the biochemical detection unit 123, or the electrolyte analysis unit 125.

The processing section 13 receives the information such as the measurement results notified from the mechanism of the analytic operation section 20, performs data processing corresponding to the analysis type, and calculates and stores analysis result information. Along with that, the processing section 13 notifies the operation section 30 of the analysis result information requested by the user. The operation section 30 displays the analysis result information on the screen of the display device 32.

After entering the multiple pieces of analysis request information, the user presses a start button in the screen, for example. This triggers the automatic analyzer 1 to start the analysis operation. Upon the start, the control section 10 prioritizes the above multiple pieces of analysis request information based on the score table 50, and performs assignment (in other words, reservation) of the information to the analysis processing. Subsequently, the processing section 13 performs the analysis processing using the analytic operation section 20 in the processing order according to the analysis reservation information.

### [Information Elements]

In the embodiment, description is now given on information, elements, and viewpoints to be considered and used for prioritization involved in the analysis request information and determination of the analysis processing order. For the sake of explanation, such information, elements, and viewpoints may be collectively referred to as information elements.

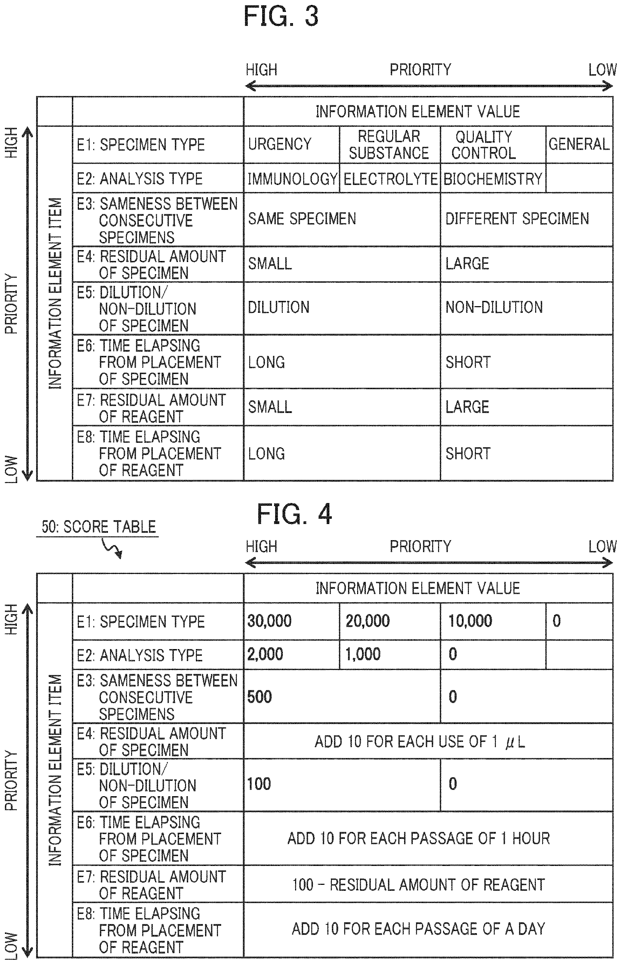

Fig. 3 shows a table summarizing examples of information elements to be considered and used for prioritization involved in the analysis request information and determination of the analysis processing order in the automatic analyzer 1 of the embodiment. In the table of Fig. 3, multiple information element items are shown in the vertical rows, and values (multiple values) for each information element are shown in the horizontal direction. Multiple information element items are illustrated with signs such as sign E1 for identification. The items of the multiple information elements include E1: Sample type, E2: Analysis type, E3: Sameness between consecutive samples, E4: Sample residual amount, E5: Dilution/non-dilution of sample, E6: Time elapsing from sample placement, E7: Reagent residual amount, and E8: Time elapsing from reagent placement. The "Nth information" in Fig. 2 corresponds to these information elements.

The sample type E1 indicates a sample type, and has four values of "urgent", "standard material", "quality control", and "general", for example. Note that the value may be expressed as 0, 1, 2, 3, ···, as appropriate.

The analysis type E2 indicates the above test classification item, and has three values: "immunology," "electrolyte," and "biochemistry."

The sameness between consecutive samples E3 is an item that indicates, when two samples, i.e., a former sample (in other words, first sample) and a latter sample (in other words, a target sample being a second sample), are consecutive, whether the latter sample is the same as or different from the former sample, and has two values: "same sample" and "different sample".

The sample residual amount E4 indicates the residual amount of a sample (target sample) in the sample container, showing a case of categorizing the amount into two values, "large" and "small", for example.

The dilution/non-dilution of sample E5 indicates a practice state of dilution of a sample (target sample), showing a case of categorizing the state into two values, "dilution" and "non-dilution".

The time elapsing from sample placement E6 indicates time elapsing from placing a sample (target sample), showing a case of categorizing the time into two values, "long" and "short", for example. The target sample refers to the same sample as a sample being an analysis target. Placement of a sample refers to placement of a sample container, in which the sample has been placed, on the sample disc 102, etc.

The reagent residual amount E7 is the residual amount of a reagent (target reagent) in a reagent container, showing a case of categorizing the amount into two values, "large" and "small", for example.

The time elapsing from reagent placement E8 indicates time elapsing from placing a reagent (target reagent), showing a case of categorizing the time into two values, "long" and "short", for example. The target reagent refers to the same reagent as a reagent used for a sample to be analyzed. Placement of a reagent refers to placement of a reagent container, in which the reagent has been placed, in the reagent storage 105.

In the table of Fig. 3, using the concept of priority (first priority), the multiple information element items in the vertical direction are arranged along height of the first priority. The first priority is a priority between the information elements. Further, in the table of Fig. 3, using the concept of priority (second priority), values of the information elements in the horizontal direction are arranged along height of the second priority. The second priority is a priority between the values of the information elements.

The sample type E1 is provided to control the priority order/priority of the analysis processing according to the sample type. For example, if the value of the sample type in the analysis request information is "urgent", the priority order is set higher, and if it is "general", the priority order is set lower. Higher priority/priority order refers to raising the order of analysis processing of the target sample.

The analysis type E2 is provided to control the priority order/priorityof analysis processing according to the analysis type. The automatic analyzer 1 has different operating cycles (in other words, cycle times) for each of the analysis types, for example, (details are described later). The automatic analyzer 1 is configured with different operating cycles for each analysis type E2 (immunology, electrolyte, and biochemistry), in correspondence to differences in flow between the analysis types described above. For example, cycle time of immunity analysis is longer than cycle time of biochemical analysis . In other words, analysis processing corresponding to an analysis request of one sample occupies a longer cycle time for immunity analysis . Therefore, when sample analysis request information is assigned to the cycle time of the analysis type, it is more difficult to assign the information to the cycle time of immunological analysis than to the cycle time of biochemical analysis.

An example of prioritization using the above information elements therefore includes prioritizing multiple pieces of analysis request information for multiple samples in correspondence to the case where the operation cycle depends on the analysis type. Specifically, in the example of the analysis type E2 in the table of Fig. 3, "immunology", which is relatively difficult to be assigned, is given a higher priority, and "biochemistry", which is relatively easy to be assigned, is given a lower priority.

Similarly, for other information elements, when analysis request information is assigned to analysis processing (e.g., corresponding mechanism or cycle time), a priority or priority order is controlled higher or lower based on the concept of each information element.

For the sample residual amount E4 and the reagent residual amount E7, the smaller the residual amount, the higher the priority. The reagent and the sample increasingly degrade over time after placement. For the time elapsing from sample placement E6 or the time elapsing from reagent placement E8, therefore, the priority is considered to be raised with the time longer.

In the table of Fig. 3, the multiple information element items in the vertical direction are arranged along the height of the first priority. Specifically, in this example, the priority changes from high to low in order of the sample type E1 to the time elapsing from reagent placement E8. In this example, the highest priority is given to the sample type E1, and the second highest priority is given to the analysis type E2. The sample type E1 is given the highest priority due to its large impact on customer requests and analysis accuracy. The analysis type E2 is given the second highest priority in consideration of impact on analysis time and the above-described competition between the mechanisms. The sameness between consecutive samples E3, the sample residual amount E4, the dilution/non-dilution of sample E5, and the time elapsing from sample placement E6 are sequentially given the next lower priority due to their impact on the analysis accuracy for the sample. The reagent residual amount E7 and the time elapsing from reagent placement E8 are further sequentially given a next lower priority, i.e., given the lowest priority between the information elements due to impact of the reagent as a consumable.

The automatic analyzer 1 also has a function of determining (in other words, detecting and calculating) values of predetermined information elements (for example, sample residual amount E4, reagent residual amount E7, and time elapsing from placement or the sample or reagent) handled in the score table 50, and also holds the values and information determined by the function. The details of this function are not limited, and known technical means/method can be applied to the function. As an example, the time elapsing from sample placement E6 can be determined by measuring, using a clock, time from the time point of placement of the sample container in the sample disc 102.

### [Score Table]

Fig. 4 shows an exemplary configuration of a score table 50, which is used for prioritizing analysis request information and determining the analysis processing order, corresponding to the case of using the concept of the information elements and priority in Fig. 3. In the score table 50, scores are defined in correspondence to the height of the priority in Fig. 3.

The structure of the score table 50 of Fig. 4 has scores entered in cells of the matrix in correspondence to the items and values of the multiple information elements (E1 to E8) in Fig. 3. In the score table 50, although the values of the information elements (such as value of "urgent" in Fig. 3) are omitted to avoid complexity, scores are actually preset in correspondence to the values of the information elements.

As shown in the figure, in the score table 50, items and values of information elements with higher priorities (first priority and second priority) are set to have higher scores. For example, in a row of the sample type E1, scores are set in tens of thousands of digits, such as 30000, 20000, 10000, and 0, in descending order of the second priority in correspondence to the four values in the horizontal direction. In a row of the analysis type E2 below that, scores in thousands of digits, such as 2000, 1000, and 0, are set in descending order of the second priority in correspondence to the three values in the horizontal direction. Similarly, the vertically lower the information element is, the smaller the score is set.

In a row of the sameness between consecutive samples E3, the value "same sample" is set to 500, and the value "different sample" is set to 0. Such a score is set with the idea that if the same sample appears consecutively, it will be given higher priority.

In this example, in a row of the sample residual amount E4, a score calculation formula is set according to multiple values, rather than the binary value of large/small. The calculation formula is set, for example, as "add 10 for each use of 1 µL." The sample usage has a predetermined relationship to the sample residual amount. For example, if the amount used in the analysis of a target sample is 10 µL, the score for this item is 10 × 10 = 100. When definition is made with the above binary value of large/small, the following definition may also be acceptable: For example, the sample usage is divided into binary values using a threshold, and a score X is given for the value "large" while a score Y is given for the value "small". This score calculation formula is set with the idea that the smaller the residual amount of the sample, the higher the priority.

In the row of the dilution/non-dilution of sample E5, the score is set to 100 for "dilution" and 0 for "non-dilution". Such scores are set with the idea that higher priority is given in the case of "dilution".

In this example, in the row of the time elapsing from sample placement E6, a score calculation formula is set according to multiple values, rather than a binary value of long/short. For example, the calculation formula is set as "add 10 for each passage of 1 hour." For example, if the time elapsing from placement of the target sample is 2 hr, the score for this item is 2 × 10 = 20. This score calculation formula is set with the idea that the longer the time elapsing from sample placement, the higher the priority.

In this example, in the row of the reagent residual amount E7, a score calculation formula is set according to multiple values, rather than a binary value of large/small. For example, the calculation formula is set as "100 - residual amount of reagent (%) " . For example, if the residual amount of the target reagent is 20% of the maximum amount, the score for this item is 100 - 20 = 80. This score calculation formula is set with the idea that the smaller the residual amount of the reagent used in the analysis of the target sample, the higher the priority.

In this example, in the row of the time elapsing from reagent placement E8, a score calculation formula is set according to multiple values, rather than a binary value of long/short. For example, the calculation formula is set as "add 10 for each passage of a day." This score calculation formula is set with the idea that the longer the time elapsing from reagent placement, the higher the priority.

In the score table 50 of Fig. 4, for the first priority between the information element items in the vertical direction, the score is set to vary according to height at the digit level. When a large difference is provided in the first priority between the information elements in the vertical direction, the digit level of the score group for each information element is set to vary as in this example. As a result, briefly, an information element with a higher first priority has a larger score and has a greater impact on prioritization. Furthermore, according to the horizontal value within each information element item, the score is also adjusted to be higher or lower within the digit so as to affect prioritization. In this way, the score is set to vary greatly at the digit level, for example, according to the first priority between the information elements, making it possible to prevent operation with unintended priority, for example.

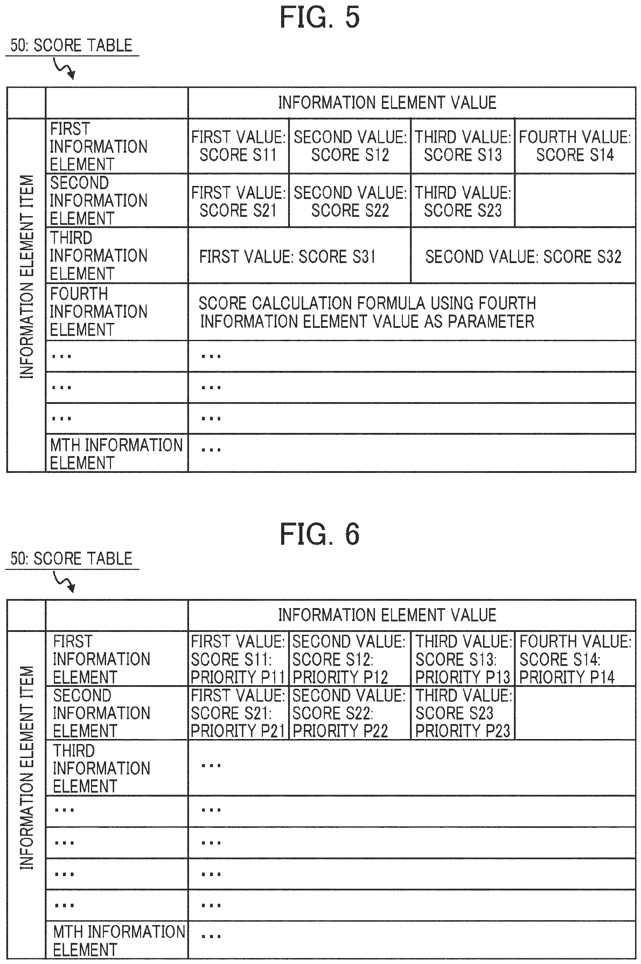

Fig. 5 shows an exemplary configuration of the score table 50 more abstracted than that of Fig. 4. The score table 50 can be implemented in a data structure such as a table. The multiple vertical rows of the score table 50 indicate items of multiple information elements, and the horizontal columns thereof indicate values for each information element item. Each cell in the matrix has a score or a score calculation formula according to the value of the information element. For example, the first information element item has values from a first value to a fourth value, and a score is set for each value. For example, the score for the first value is score S11, and the score for the second value is score S12. For example, in the fourth information element item, a score calculation formula is set with the value of the fourth information element as a parameter. A prescribed format other than the score calculation formula may include, for example, a computer program implemented with an algorithm using values of information elements as a parameter.

In the score table 50 of Fig. 5, the above priorities (first priority and second priority) are not explicitly held, but are reflected in the score or the set value of the calculation formula.

Fig. 6 shows a score table 50 in a modification, showing a case where the above-described priorities (first priority and second priority) are explicitly set and held as one data item. For example, in the item of the first information element, a score (such as S11) and priority (such as P11) are set for each value in the horizontal direction. Similarly, in the score table of another configuration example, the data item of the first priority may be explicitly set and held for each information element item in the vertical direction.

In the modification, the following method may be used: when a score is calculated for each piece of analysis request information, the score is calculated using a standard score and a priority value according to the value of the information element. For example, first, a standard score (a score that does not reflect priority), which corresponds to the value of the information element, is referenced. Second, the standard score is multiplied by a priority value corresponding to the value of the information element as a value of a weight or a coefficient, for example. As a result, a score reflecting the priority is produced.

A score table that holds priority as above is also useful when a user can edit and set the score table as in modification 1 described later, for example.

### [Processing Flow]

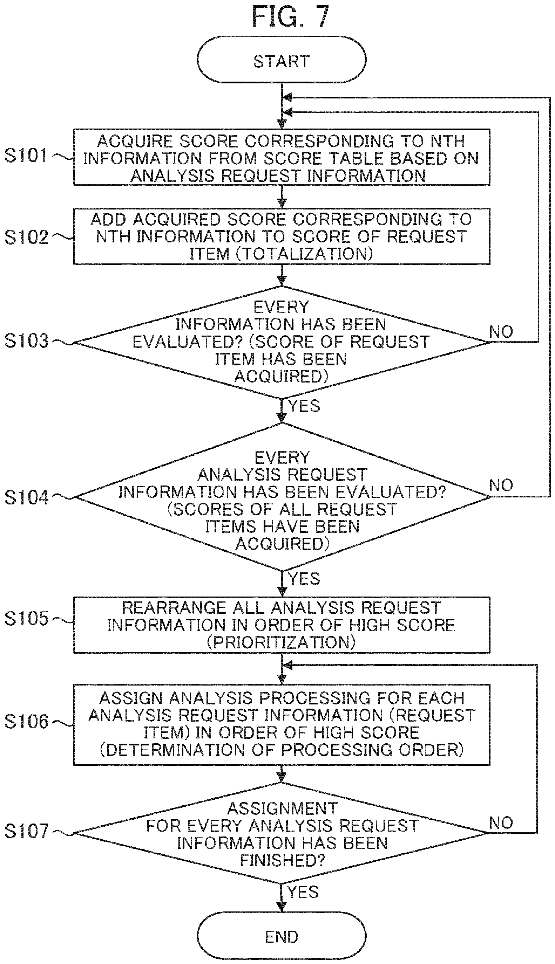

Fig. 7 shows a main processing flow by the control section 10 (corresponding processor) in the automatic analyzer 1 of the embodiment, which relates to prioritizing the analysis request information and determining the analysis processing order. The flow of Fig. 7 has steps S101 to S107. This processing flow is executed, for example, for multiple pieces of analysis request information received in a certain period. In the drawing, N of the Nth information indicates a number for identifying an item among the multiple information elements in the score table 50 described above.

The acquisition section 11 acquires a score one by one from the score table 50 for one or more information elements (N = 1 to M) related to requested items of the sample analysis request information (items such as inspection, analysis, and measurement in the specified analysis type), and totalizes all the acquired scores in units for each piece of analysis request information. For example, for one piece of analysis request information, respective scores, which correspond to the values of the above eight information elements E1 to E8, are acquired, and the eight scores are summed. In the embodiment, the totalization is defined as, but not limited to, the sum by addition. In the modification, the totalization may be performed, for example, by performing weighted addition of the scores for each information element using a weight corresponding to the first priority between the information elements.

When the acquisition section 11 completes the totalization of scores for the values of all information elements of a certain analysis request information (that is, calculation of scores for each request item), the acquisition section 11 similarly totalizes the scores for the next analysis request information in order of reception. The acquisition section 11 acquires multiple scores for all multiple pieces of analysis request information.

The determination section 12 determines the order of analysis processing by the processing section 13 for the multiple pieces of analysis request information based on the scores acquired by the acquisition section 11. In other words, the determination section 12 determines the assignment of each piece of analysis request information to analysis processing (mechanism, cycle time, or the like).

The determination section 12 reserves analysis processing of the processing section 13 such that the analysis processing is started with the highest priority for the analysis request information having the highest score among the multiple scores of the multiple pieces of analysis request information. Similarly, the determination section 12 reserves analysis processing of the processing section 13 such that analysis processing is started with priority for each piece of analysis request information in descending order of scores. The control section 10 repeats such processing and operations in the same way until completion of the analysis reservations for all of the multiple pieces of analysis request information received at that time. The control section 10 performs the same processing if it receives new analysis request information at another time.

The flow of Fig. 7 is an implementation example of the above processing. First, in step S101, the acquisition section 11 of the control section 10 acquires a score corresponding to the Nth information (value of the information element) related to the analysis request information from the score table 50 of the storage section 41 based on the received analysis request information. N is 1 at first and is incremented by 1 for each repeat of a loop up to the number of information element values (M) (N = 1 to M) . In step S102, the acquisition section 11 adds the acquired score corresponding to the Nth information to the score of each piece of analysis request information (in request item unit). In step S103, the acquisition section 11 checks whether all the information elements related to the analysis request information have been evaluated, in other words, whether the scores of all items have been acquired from the score table 50, and if the evaluation has not yet been finished (No), the processing returns to step S101 and processing is repeated in the same way. If the score totalization of all information elements is finished (Yes), the processing advances to step S104.

In step S104, the acquisition section 11 checks whether all pieces of analysis request information have been evaluated, or in other words, whether multiple scores for all pieces of analysis request information (corresponding request items) have been acquired, and if the evaluation has not been finished yet (No), the processing returns to step S101 and processing is repeated in the same way for the next analysis request information. If all evaluations have been finished, that is, if scores for all pieces of analysis request information have been acquired (Yes), the processing advances to step S105.

In step S105, the determination section 12 rearranges, in descending order of scores, all of the multiple pieces of analysis request information received in a certain period. In other words, such processing up to this rearrangement corresponds to prioritization (especially prioritization as the first step) of the multiple pieces of analysis request information.

In step S106, the determination section 12 assigns each piece of analysis request information (corresponding request item) to analysis processing (mechanism, cycle time, etc.) of the processing section 13 in descending order of scores (order after the rearrangement). Such assignment (in other words, reservation) corresponds to determining the processing order for each request item. This corresponds to primary determination or provisional reservation in the middle of the processing flow, and the processing order may vary as appropriate between the request items according to the processing order assignment of subsequent another request item.

In step S107, the determination section 12 checks whether the above assignment has been finished for all pieces of the analysis request information, and if it has not been finished yet (No), the processing returns to step S106 and processing is repeated in the same way. If the processing has been finished (Yes), such finish corresponds to completion of prioritization (particularly, determination of actual processing order and analysis reservation as the second step) of the multiple pieces of analysis request information by the processing up to this step. This is the end of the flow of Fig. 6. From the perspective of the processing section 13, analysis processing can be started in the processing order according to the prioritized analysis reservation information after the above flow processing.

### [Analysis Request Information]

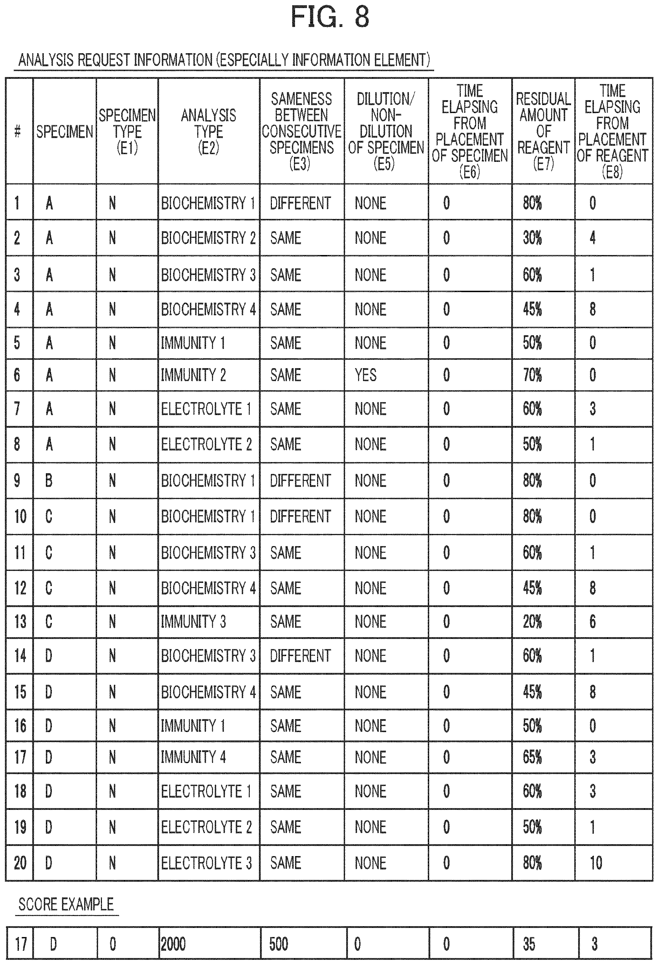

Fig. 8 shows, in a table, a specific example of multiple pieces of analysis request information (particularly information elements related to them) entered by a user on a screen during a certain period. A specific example of the above processing is now described. The table of Fig. 8 shows, in columns, number (#), sample, sample type, analysis type, sameness between consecutive samples, dilution/non-dilution of sample, time elapsing from sample placement, reagent residual amount, and time elapsing from reagent placement. The number (#) indicates input order of analysis request information and line number. The sample column indicates ID to identify the sample. The columns from the sample type to the time elapsing from reagent placement correspond to the information element items (E1, E2, E3, E5, E6, E7, E8) in the above score table 50. In this example, the sample residual amount E4 is not considered.

Thus, in this example, 20 pieces of analysis request information in the input order of #1 to #20 exist at almost the same timing. In this example, every sample type E1 is normal (N). For the analysis type E2, there are the above three broad categories (immunology, electrolyte, and biochemistry), and in this example, there are more detailed categories, such as "biochemistry 1" and "biochemistry 2". For example, even within the same category of biochemical analysis, the category is divided into "Biochemistry 1" and "Biochemistry 2" according to differences in details of the analysis. For example, although "Biochemistry 1" and "Biochemistry 2" are both performed using the biochemical analysis mechanism 21 in Fig. 2, the details of the measurement, etc. are different therebetween.

For the sameness between consecutive samples E3, for example, the sample = A in the request # = 1 is the first request and there is no previous sample to compare with, and is thus specified as "different sample". The sample= A of the next request # = 2 is the same as the previous sample # = 1, and is thus specified as "same sample". For example, the sample = B of the request # = 9 is different from the previous sample = A of # = 8, and is thus specified as "different sample". The time elapsing from sample placement E6 is less than 1 hr in every sample in this example and is thus 0 in each sample.

### [Score and Priority Order]

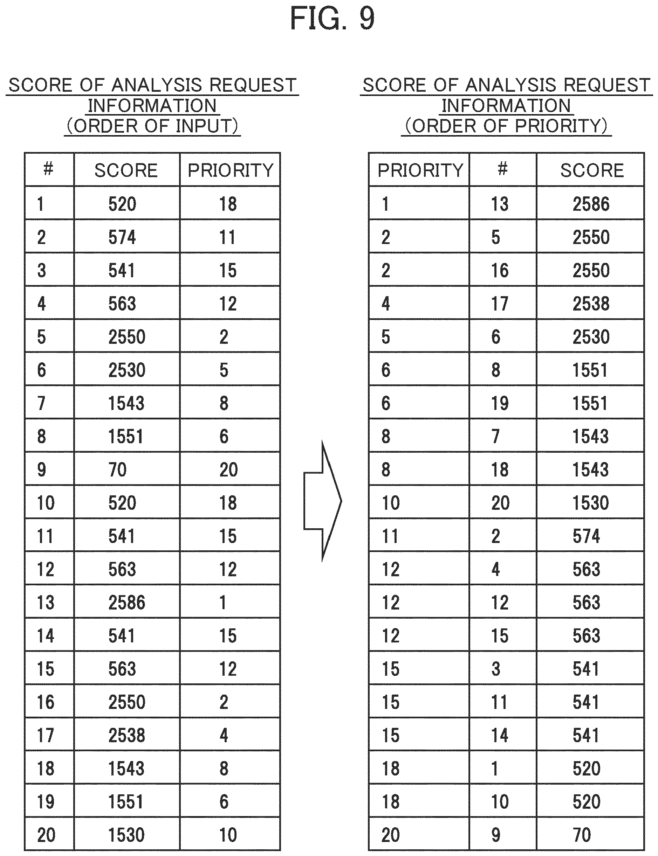

Fig. 9 shows an example of scores for each request item acquired from the score table 50 (Fig. 4) based on the multiple (20) pieces of analysis request information shown in Fig. 8. The table on the left shows scores in order of input (#), and the table on the right shows scores rearranged in order of high score by the determination section 12. In other words, this score order corresponds to the priority order (first step prioritization) related to the analysis processing. In the table on the left, the "priority" column shows a numerical value starting from 1 in descending order of scores, and if the scores are the same, the numerical values are assigned to the same priority. The table on the right shows the contents rearranged according to the numerical values of "priority order".

In this example, as shown in the table on the right, priority order = 1 is given to the analysis request information of input order (#) = 13. Priority order = 2 is given to two pieces of analysis request information of # = 5 and # = 16. The 20 pieces of analysis request information are as follows in order of priority: 13, (5, 16), 17, 6, (8, 19), (7, 18), 20, 2, (4, 12, 15), (3, 11, 14), and (1, 10), 9. Figures in the parentheses () are in the same order. Two or more pieces of analysis request information that are given the same priority may be processed in the following assignment, for example, in ascending order of the number of input order.

In an example of calculating the score for each piece of analysis request information, the following is given for the analysis request information of # = 17. The analysis request information of # = 17 shows sample = D, sample type E1 = "Normal (N)," analysis type E2 = "Immunity 4", sameness between consecutive samples E3 = "Same", dilution/non-dilution of sample E5 = "Non-dilution", time elapsing from sample placement E6 = 0, reagent residual amount E7 = "65%", and time elapsing from reagent placement E8 = 3. As a result, the score of the analysis request information of # = 17 is {0, 2000, 500, 0, 0, 35, 3} for each information element item, and when totalized (summed), the score is 2538.

### [Comparative Example: Analysis Reservation]

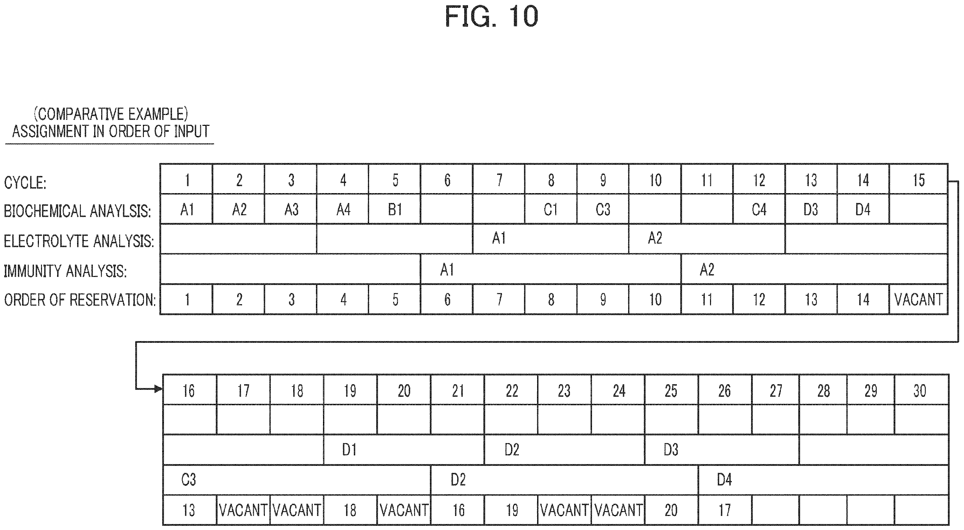

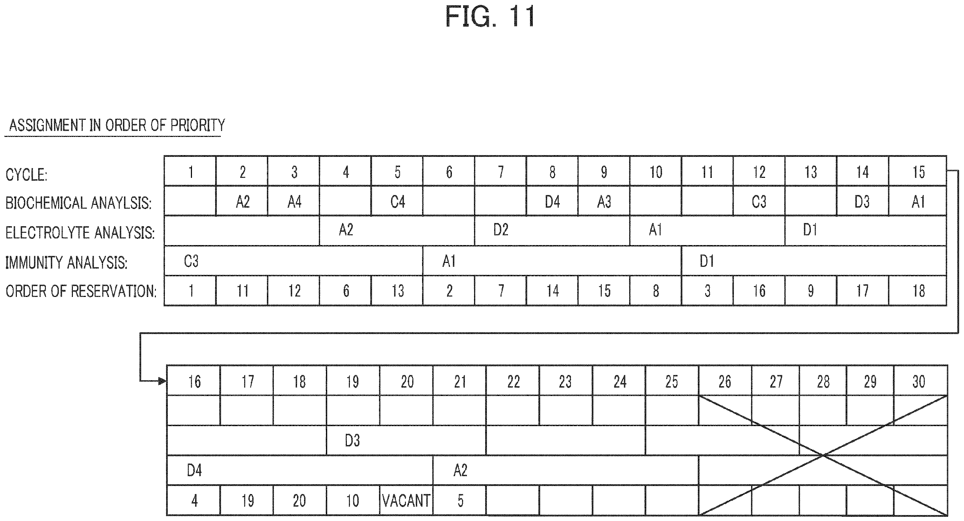

Figs. 10 and 11 are schematic illustrations of assignment (in other words, reservation) of multiple (20) pieces of analysis request information and scores in the above Figs. 8 and 9 to the analysis processing. The table of Fig. 10 shows an example of assignment according to input order as a comparative example, and the table of Fig. 11 shows an example of assignment according to priority order in the embodiment.

First, in the table of Fig. 10, the horizontal axis corresponds to cycle time (operation period) in the time direction, and one cell corresponds to one cycle time unit (a unit that occupies a predetermined time). On the vertical axis, the first row shows identification information of cycle time. The second row shows a biochemical analysis cycle when the analysis type is biochemistry. The third row shows an electrolyte analysis cycle when the analysis type is electrolyte. The fourth row shows an immunity analysis cycle when the analysis type is immunity. The fifth row indicates the order of assignment/reservation of analysis request information (corresponding request item).

Each cell is left vacant if there is no assignment. If there is assignment, the cell has information on assigned sample and analysis type. For example, in the row of the biochemical analysis cycle, the notation "A1" indicates that analysis processing of a request item of sample= A and analysis type = "Biochemistry 1" has been assigned. The analysis processing of "A1" is assigned to a cycle time for one cell in the biochemical analysis cycle. Similarly, for example, in the row of the electrolyte analysis cycle, the notation "A1" indicates that analysis processing of a request item of sample = A and analysis type = "Electrolyte 1" has been assigned. This "A1" analysis processing is assigned to cycle times for 3 cells in the electrolyte analysis cycle. Similarly, for example, in the row of the immunity analysis cycle, the notation "A1" indicates that analysis processing of a request item of sample = A and analysis type = "Immunity 1" has been assigned. The analysis processing of "A1" is assigned to cycle times for 5 cells in the immunity analysis cycle.

As described above, analysis for each analysis type (biochemistry, electrolyte, immunity) has a different cycle time, and in a specific example, as shown in the figure, the cycle time is one (e.g., 10 sec) for biochemical analysis, three (e.g., 30 sec) for electrolyte analysis, and five (e.g., 50 sec) for the immunity analysis. Opportunities of reservation of analysis are more (in other words, assignment is easier) for an analysis type with a shorter cycle time, and thus the opportunities are less (in other words, assignment is more difficult) in order of biochemistry, electrolyte, and immunity. In the embodiment shown in Fig. 11 described later, therefore, the analysis request information is controlled by the above score such that the longer the cycle time, the higher the priority/priority order of the analysis type.

In the comparative example, the processor sequentially assigns analysis processing for each piece of sample analysis request information to the analysis processing cycle as shown in Fig. 10 according to the input order (#) in the table of Fig. 8. First, the request item # = 1 shows sample = A and analysis type = "Biochemistry 1", and is assigned as "A1" to cycle time 1 of the biochemical analysis cycle. Correspondingly, "1" is written in the reservation order row. Subsequently, the request item # = 2 shows sample= A and analysis type = "Biochemistry 2", and is assigned as "A2" to cycle time 2 of the biochemical analysis cycle. Correspondingly, "2" is written in the reservation order row. Similarly, request items # = 3 and # = 4 are assigned to cycle times 3 and 4 as "A3" and "A4", respectively.

Subsequently, the request item # = 5 shows sample= A and analysis type = "immunity 1", and is assigned as "A1" to cycle times 6 to 10 for 5 cells in the immunity analysis cycle. At this time, since "A1" of "Biochemistry 1" has already been assigned to the cycle time 1, "A1" of "Immunity 1" is not assigned at the same time, but is assigned to cycle time 6, which is vacant after the cycle time 1. Subsequently, the request item # = 6 shows sample= A and analysis type = "immunity 2", and is assigned as "A2" to cycle times 11 to 15 for 5 cells in the immunity analysis cycle.

Subsequently, the request item # = 7 shows sample= A and analysis type = "Electrolyte 1", and is assigned as "A1" to cycle times 7 to 9 for 3 cells in the electrolyte analysis cycle. Subsequently, the request item # = 8 shows sample= A and analysis type = "Electrolyte 2", and is assigned as "A2" to cycle times 10 to 12 for 3 cells in the electrolyte analysis cycle.

Subsequently, the request item # = 9 shows sample= B and analysis type = "Biochemistry 1", and is assigned as "B1" to cycle time 5 of the biochemical analysis cycle. Subsequently, the request item # = 10 shows sample= C and analysis type = "Biochemistry 1", and is assigned as "C1" to cycle time 8 of the biochemical analysis cycle. At this time, although cycle times 6 and 7 subsequent to "B1" of the biochemical analysis cycle are vacant, since the cycle times have been used for "A1" of the electrolyte analysis and "A1" of the immunity analysis cycle at the same time, the subsequent cycle time 8 is used. Subsequently, the request item # = 11 shows sample= C and analysis type = "Biochemistry 3", and is assigned as "C3" to cycle time 9 of the biochemical analysis cycle. Subsequently, the request item # = 11 shows sample= C and analysis type = "Biochemistry 4", and is assigned as "C4" to cycle time 12 of the biochemical analysis cycle. At this time, similarly to the above, since the cycle times 10 and 11 have been used for other analysis types, the cycle time 12 is used. Subsequently, the request item # = 13 shows sample= C and analysis type = "Immunity 3", and is assigned as "C3" to cycle times 16 to 20 for 5 cells in the immunity analysis cycle.

Subsequently, the request item # = 14 shows sample= D and analysis type = "Biochemistry 3", and is assigned as "D3" to cycle time 13 of the biochemical analysis cycle. Subsequently, the request item # = 15 shows sample= D and analysis type = "Biochemistry 4", and is assigned as "D4" to cycle time 14 of the biochemical analysis cycle.

Subsequently, the request item # = 16 shows sample= D and analysis type = "Immunity 1", and is assigned as "D1" to cycle times 21 to 25 for 5 cells in the immunity analysis cycle. Subsequently, the request item # = 17 shows sample= D and analysis type = "Immunity 4", and is assigned as "D4" to cycle times 26 to 30 for 5 cells in the immunity analysis cycle.

Subsequently, the request item # = 18 shows sample= D and analysis type = "Electrolyte 1", and is assigned as "D1" to cycle times 19 to 21 for 3 cells in the electrolyte analysis cycle. Subsequently, the request item # = 19 shows sample= D and analysis type = "Electrolyte 2", and is assigned as "D2" to cycle times 22 to 24 for 3 cells in the electrolyte analysis cycle. Subsequently, the request item # = 20 shows sample= D and analysis type = "Electrolyte 3", and is assigned as "D3" to cycle times 25 to 27 for 3 cells in the electrolyte analysis cycle.

The above assignment of 20 pieces of analysis request information results in the state of the table of Fig. 10. A total of 1 to 30 cycle times are used in the assignment of the comparative example. In the reservation order row, a location marked as "vacant" indicates that there is no assignment with that cycle time as a start cycle.

### [Embodiment: Analysis Reservation]

The assignment in the embodiment is now described with reference to Fig. 11. The determination section 12 assigns each piece of analysis request information (corresponding request item) to analysis operation cycle of each mechanism based on the priority order determined based on the score table 50. In time series, the head part of an analysis request for any one of analysis types is assigned to a certain cycle time.

The control section 10 (processor) sequentially assigns analysis processing for each piece of sample analysis request information to analysis processing cycle as shown in Fig. 11 (similar to Fig. 10) according to the priority order in the table on the right of Fig. 9. First, the request item with priority order = 1 (# = 13) shows sample = C and analysis type = "Immunity 3", and is assigned as "C1" to cycle times 1 to 5 for 5 cells in the immunity analysis cycle. Correspondingly, "1" is written in the reservation order row. Subsequently, the request items with priority order = 2 (# = 5, 16) include two request items respectively showing sample = A and analysis type = "Immunity 1", and sample = D and analysis type = "Immunity 1". The request item # = 5 is assigned as "A1" to cycle times 6 to 10 for 5 cells in the immunity analysis cycle. The request item # = 16 is assigned as "D1" to cycle times 11 to 15 for 5 cells in the immunity analysis cycle.

Subsequently, the request item with priority order = 4 (# = 17) shows sample = D and analysis type = "Immunity 4", and is assigned as "D4" to cycle times 16 to 20 for 5 cells in the immunity analysis cycle. Subsequently, the request item with priority order = 5 (# = 6) shows sample = A and analysis type = "Immunity 2", and is assigned as "A2" for cycle times 21 to 25 for 5 cells in the immunity analysis cycle.

Subsequently, the request items with priority order = 6 (# = 8, 19) include two request items respectively showing sample = A and analysis type = "Electrolyte 2", and sample = D and analysis type = " Electrolyte 2". The request item of # = 8 is assigned as "A2" to cycle times 4 to 6 for 3 cells in the electrolyte analysis cycle. At this time, since the cycle time 1 has been used for immunity analysis, the later cycle time 4 is used. The request item of # = 19 is assigned as "D2" to cycle times 7 to 9 for 3 cells in the electrolyte analysis cycle. Subsequently, the request items with priority order = 8 (# = 7, 18) include two request items respectively showing sample = A and analysis type = "Electrolyte 1", and sample = D and analysis type = "Electrolyte 1". The request item of # = 7 is assigned as "A1" to cycle times 10 to 12 for 3 cells in the electrolyte analysis cycle. The request item of # = 18 is assigned as "D1" to cycle times 13 to 15 for 3 cells in the electrolyte analysis cycle.

Subsequently, the request item with priority order = 10 (# = 20) shows sample = D and analysis type = "Electrolyte 3", and is assigned as "D3" to cycle times 19 to 21 for 3 cells in the electrolyte analysis cycle. At this time, since the cycle time 16 has been used for immunity analysis, the later cycle time 19 is used.

Subsequently, the request item with priority order = 11 (# = 2) shows sample = A and analysis type = "Biochemistry 2", and is assigned as "A2" to cycle time 2 of the biochemical analysis cycle. Subsequently, the request items with priority order = 12 (# = 4, 12, 15) include three request items respectively showing sample = A and analysis type = "Biochemistry 4", sample = C and analysis type = "Biochemistry 4", and sample = D and analysis type = "Biochemistry 4". The request item of # = 4 is assigned as "A4" to cycle time 3 of the biochemical analysis cycle. The request item of # = 12 is assigned as "C4" to cycle time 5 of the biochemical analysis cycle. The request item of # = 15 is assigned as "D4" to cycle time 8 of the biochemical analysis cycle.

Subsequently, the request items with priority order = 15 (# = 3, 11, 14) include three request items respectively showing sample = A and analysis type = "Biochemistry 3", sample = C and analysis type = "Biochemistry 3", and sample = D and analysis type = "Biochemistry 3". The request item of # = 3 is assigned as "A3" to cycle time 9 of the biochemical analysis cycle. The request item of # = 11 is assigned as "C3" to cycle time 12 of the biochemical analysis cycle. The request item of # = 14 is assigned as "D3" to cycle time 14 of the biochemical analysis cycle.

Subsequently, the request items with priority order = 18 (# = 1, 10) include two request items respectively showing sample = A and analysis type = "Biochemistry 1", and sample = C and analysis type = "Biochemistry 1". The request item of # = 1 is assigned as "A1" to cycle time 15 of the biochemical analysis cycle. The request item of # = 10 is assigned as "C1" to cycle time 17 of the biochemical analysis cycle. Subsequently, the request item with priority order = 20 (# = 9) shows sample = B and analysis type = "Biochemistry 1", and is assigned as "B1" to cycle time 18 of the biochemical analysis cycle.

The above assignment of 20 pieces of analysis request information results in the state of the table of Fig. 11. A total of 1 to 25 cycle times are used in the assignment of the embodiment. The total cycle time for 25 cells is shorter than the total cycle time for 30 cells in the comparative example. In other words, the embodiment is more efficient in assignment of the analysis processing than the comparative example.

In the comparative example (Fig. 10), since the processing is performed simply in order of input, for example, in comparison between the biochemical analysis cycle "A1" and the immunity analysis cycle "A1", the biochemical analysis "A1" is assigned earlier (cycle time 1), i.e., the immunity analysis "A1" is assigned later (cycle time 6) . In contrast, in the embodiment (Fig. 11), with respect to the analysis type E2, the immunity analysis has a higher priority and a larger score than the biochemical analysis. In the embodiment, therefore, for example, in comparison between the biochemical analysis cycle "A1" and the immunity analysis cycle "A1", the immunity analysis "A1" is assigned earlier (cycle time 6), i.e., the biochemical analysis "A1" is assigned later (cycle time 15) .

For example, focusing on the biochemical analysis "C4" (analysis request information of # = 12), the biochemical analysis is assigned to cycle time 12 in the comparative example (Fig. 10), while it is assigned to cycle time 5 in the embodiment (Fig. 11), resulting in earlier start time. For the information elements of the analysis request information of # = 12, for example, the reagent residual amount is relatively small (45%), and the time elapsing from reagent placement is long (8 hr) . For the analysis request information of # = 12, therefore, based on the score table 50 (Fig. 4), the score is relatively larger reflecting a decrease in reagent residual amount and reagent degradation, and the priority order correspondingly becomes higher, resulting in assignment of the information to an earlier cycle time.

For example, for immunity analysis "C3" (analysis request information of # = 13), the immunity analysis is assigned to cycle time 16 in the comparative example (Fig. 10), while it is assigned to cycle time 1 in the embodiment (Fig. 11), resulting in earlier start time. For the information elements of the analysis request information of # = 13, for example, the reagent residual amount is relatively small (20%), and the time elapsing from reagent placement is long (6 hr) . For the analysis request information of # = 12, therefore, based on the score table 50 (Fig. 4), the score is relatively larger reflecting a decrease in reagent residual amount and reagent degradation, and the priority order correspondingly becomes higher, resulting in assignment of the information to an earlier cycle time.

The above example embodiment has been described with a case where when the determination section 12 assigns multiple pieces of analysis request information to analysis processing based on the priority order of the information, it sequentially assigns the analysis request information one by one to a mechanism or cycle time according to the priority order. Not only this, but in a modification, multiple pieces of analysis request information can be assigned to mechanisms or cycle times simultaneously in parallel according to the priority order.

The above-described analysis reservation information can be confirmed on the display screen of the operation section 30 and is also saved as history information. The control section 10 causes a screen of the analysis reservation information created in the analysis reservation (for example, a screen showing information content as shown in Fig. 11) to be displayed on the display screen of the operation section 30. Further, the control section 10 can also predict time from start to end for each piece of sample analysis request information based on the analysis reservation information. For example, the control section 10 calculates predicted time, including start time, end time, and required time of the analysis processing, for each piece of sample analysis request information, and causes the calculated times to be displayed on the screen. This allows a user to see the predicted time information on the screen, which facilitates analysis operation.

### [Effects]

As described above, according to the automatic analyzer of the embodiment, for the type of the automatic analyzer with multiple-type analysis functions implemented in the automatic analyzer alone, multiple pieces of analysis request information involved in multiple-type analysis can be efficiently processed. According to the embodiment, in this type of the automatic analyzer, it is possible to minimize delays caused by competition between mechanisms used for respective analysis types, reduce time to analytical results required in clinical diagnostic operations, and suppress degradation of the sample and reagent over time.

### [Modification 1]

Usable modifications of the embodiment include the following. An automatic analyzer of a modification (referred to as modification 1) provides a function as one of user setting functions, which allows a user to edit and set a score table for the prioritization (in other words, prioritization evaluation setting information). As a result, in case of prioritizing analyses of multiple analysis types for multiple samples, it is possible to achieve the analysis processing order that satisfies demands of a user based on the score table edited and set by the user. This makes it possible to provide a more user-friendly automatic analyzer.

In the modification 1, a user (who may be a business operator) can set and edit the score table 50 on the screen of the operation section 30 as necessary. The score table 50 edited by the user can be saved in the storage section 41 in Fig. 1. The storage section 41 can store multiple score tables 50. If the storage section 41 has multiple score tables 50, the score table 50 to be applied can be selected by an instruction or setting on the screen by the user, for example. The user can appropriately use the multiple score tables 40.

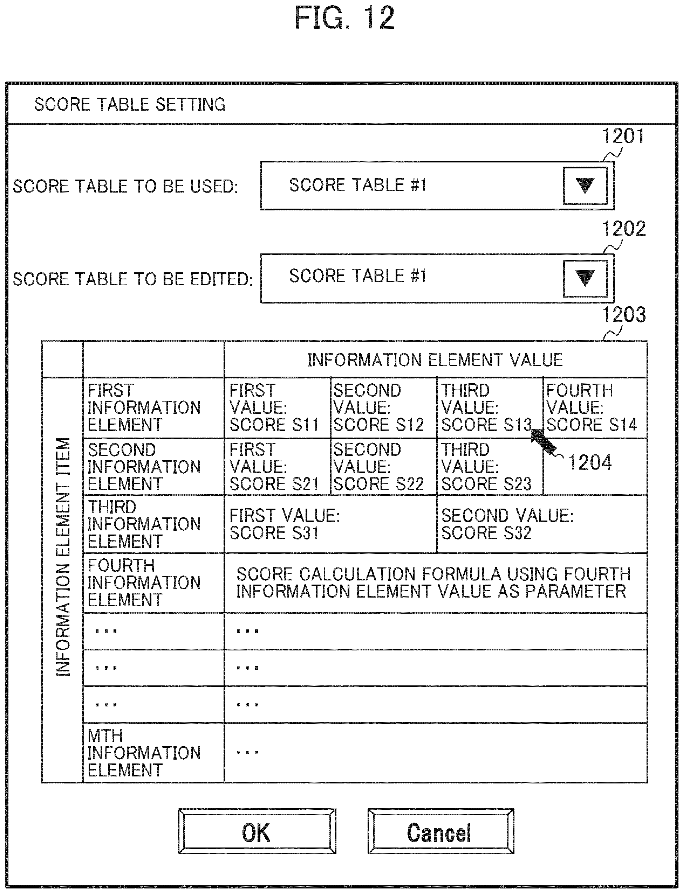

Fig. 12 shows an exemplary screen, on which the user can edit and set the score table 50, in the modification 1. The control section 10 provides a screen (for example, a web page) for editing and setting the score table 50 on the screen of the display device 32 of the operation section 30. In the screen of Fig. 12, first, a field 1201 is provided at the top so as to allow the score table 50 being normally used to be set or selected. In the lower part, there are a column 1202 for specifying the score table 50 to be edited by the user and an area 1203 for displaying the specified score table. The user can edit the value and score of the information element of a desired cell in the score table displayed in the area 1203 by operation of a mouse cursor 1204 or keyboard input, for example. Alternatively, the user can edit the score calculation formula of the information element item by changing a parameter value, for example. For example, for the sample residual amount E4 in Fig. 4, 10 in "add 10 for each use of 1 µL" can be changed to another value. After editing the score table on the screen, the user can update and save the edited score table by pressing the OK button. The user can name and save each edited score table.

In the modification 1, when the score table 50 predefined by the business operator as a default is defined as the first score table, the first score table is held as unchangeable, and a score table, which is edited and created by the user with the first score table as a base, is saved as a second score table. If there is a threshold for classifying the value of the information element, the threshold may be displayed on the screen to allow user setting.

According to the modification 1, the above editing of the score table by a user allows the user to change priority of an item and of a value of a desired information element. For example, it is possible to set a score higher so as to increase the first priority of a desired information element among multiple information elements, or to set a score higher so as to increase the second priority of a value of a desired information element. This makes it possible to adjust the score table according to user environment, user operation, or the like.

Examples of using multiple score tables according to user setting include the following. In the usage environment (for example, a hospital) of the automatic analyzer 1, the score table to be used is selected according to the date, time, department, etc. For example, a score table A is used during the day, and a score table B is used during the night. The hospital mostly deals with general outpatients during the daytime, while mostly deals with inpatients during the nighttime. The score Table A therefore gives higher priority to the sample type and analysis type corresponding to outpatients, for example. The score Table B gives higher priority to the sample type and analysis type corresponding to inpatients. Furthermore, the score table B may give a higher priority with an increase in reagent residual amount to suppress consumption of consumables or prevent consumables from running out, for example. The score Table B may give a higher priority to another sample than to the same sample for the item of the sameness between consecutive samples. Such selection of one to be used between multiple score tables may be settable by a user in advance on the screen. For example, the screen displays the date, time, and department, and the user can select and set a score table to be used for each date, time, and department.

The score table editing function as in the modification 1 can also be used for initializing, maintaining, and upgrading the score table by a business operator, for example. The business operator sets scores in the score table, taking into account various information elements and priorities. For example, using the set score table, the business operator tries to make an analysis reservation as an experiment, with various pieces of analysis request information as input, and checks whether the results (for example, required time, etc.) are preferable. The business operator adjusts the score table if the results are not preferable. In this way, a preferable score table can be established.

### [Modification 2]

The following is also possible as another modification (referred to as modification 2) . In the modification 2, as in the modification 1, a user can edit and set the score table 50. In particular, the modification 2 allows operation of changing priorities for multiple information elements in the score table 50, including the first priority between items in the vertical direction and the second priority between values in the horizontal direction. In the modification 2, in case of editing the score table 50, a user does not directly enter and set a score value into a cell, but performs an operation to change the priority on an item or value of the information element. In response to the operation, the control section 10 automatically determines a set value of the score.

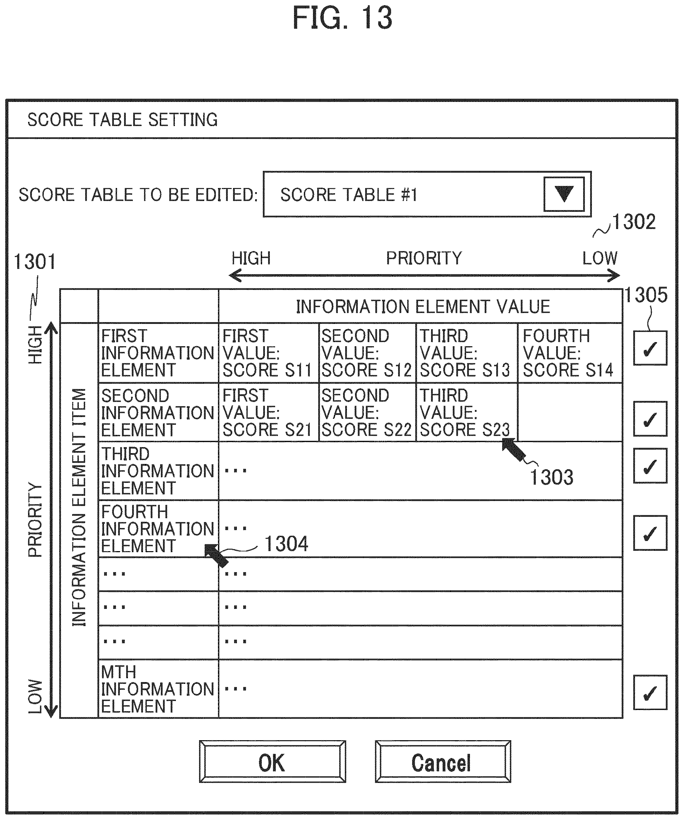

Fig. 13 shows an exemplary screen of an editing function of the score table in the modification 2. A configuration of the screen of Fig. 13 is roughly the same as that of Fig. 12, but is different from that in first priority display 1301, second priority display 1302, etc. In the screen, for example, if there are multiple value cells for an information element item, a user can perform operation to rearrange the value cells in the horizontal direction. For example, the user uses a mouse cursor 1303 to select a desired cell and move it left or right. This changes arrangement of the multiple value cells for the information element item. Such rearrangement corresponds to changing the second priority of the value of the information element.

In addition, this screen allows rows of multiple information elements to be rearranged in the vertical direction. For example, a user uses a mouse cursor 1304 to select a desired information element item and move it up or down. This changes arrangement of the rows of the multiple information elements. Such rearrangement corresponds to changing the first priority between the information elements.

For example, if a user wants to give higher priority to biochemistry than immunology for the analysis type E2 (Fig. 3), the user selects a biochemistry cell and moves the cell to the position of the immunology cell on the left. Accordingly, the immunology cell and electrolyte cell are each shifted one position to the right. As a result, the biochemistry cell has the highest priority in the analysis type E2. Depending on the second priority, set values for scores of the respective elements of biochemistry, immunology, and electrolyte are determined by automatic calculation. The automatic calculation can be enabled in the following manner, for example. In one method, with respect to a basic score setting value initialized in advance, the control section 10 automatically calculates an updated set value of a score by weighted addition that reflects the weight according to priority, for example. In another method, the score is determined according to a position of the value (corresponding cell) for an information element item. For example, in Fig. 4, for analysis type E2, the scores are as follows: Immunology = 2000, electrolyte = 1000, and biochemistry = 0. When the biochemistry cell is moved to the leftmost immunology cell position, the changed scores are as follows: Biochemistry = 2000, immunology = 1000, and electrolyte = 0. Similar operations and calculations can be applied to the first priority between the information elements in the vertical direction.

This screen has check boxes 1305 for each information element on the right side of the score table area. On this screen, for each of the information element items (E1 to E8 above) that are prepared in advance in the default score table, the user can set whether to use the item (i.e., whether to reflect the item in the prioritization) by checking or unchecking the check box 1305. By default, all information element items are on (checked) . For example, if the user wants to temporarily prevent a certain information element item from being reflected in the prioritization, the user unchecks the corresponding check box 1305. As a result, that information element item is no longer reflected in the score of the analysis request information.

According to the modification 2, even if a user is a laboratory technician with little knowledge and experience and is less likely to consider and determine a suitable detailed score, the user can easily create a user-set score table by simply manipulating priority of the item or value of the information element in editing of the default score table.

In a related-art example, when setting information using a decision tree is used to prioritize analysis requests, and when a variety of information elements are dealt with, the structure of the decision tree becomes complex, and it is complicated and time-consuming to change the setting information of the decision tree by design change or user setting. On the other hand, when using a score table as in the embodiment or each modification, it is easier to perform design change and user setting while dealing with a variety of information elements.

Although the invention has been specifically described according to the embodiment hereinbefore, the invention should not be limited thereto, and various modifications can be made within the scope without departing from the gist of the invention. Although the embodiment has been described with a case of dealing with three analysis types of biochemistry, immunity, and electrolyte, the invention is not limited to this. The invention can be similarly applied to other analysis types, such as coagulation analysis and mass spectrometry.

In the embodiment, although the calculation of the score for each piece of analysis request information has been made as addition of the score for each information element, this is not limitative. The score calculation may be performed using four arithmetic operations, functions, etc. For example, the scores for each piece of analysis request information may be totalized by multiplying the scores for each information element. In the score table, a score calculation formula for each piece of analysis request information may be defined, or the score calculation formula for each piece of analysis request information may be editable on a user setting function screen.

### List of Reference Signs

- 1:: automatic analyzer
- 10:: control section
- 11:: acquisition section
- 12:: determination section
- 13:: processing section
- 20:: analytic operation section
- 30:: operation section
- 40:: storage device
- 41:: storage section
- 50:: score table

## Claims

1. An automatic analyzer comprising:
a mechanism for analyzing a specimen with respect to each of multiple analysis types;
a processor which receives specimen analysis request information based on an input by a user, and controls the mechanism to execute an analytical operation in accordance with a designated analysis type for processing the corresponding analysis; and
a storage device which stores evaluation setting information for prioritizing the analysis request information that involves an order of the analysis, wherein:
the evaluation setting information has an evaluation value that reflects priority in the analysis order set on each of multiple information elements relating to the analysis;
the multiple information elements include at least a specimen type and the analysis type; and
the processor calculates the evaluation value set on each of the received multiple analysis request information based on the evaluation setting information, and determines an order of priority for assignment of the analysis processing in accordance with the evaluation value.

2. The automatic analyzer according to claim 1, wherein the processor determines an order of the analysis processing of the multiple analysis request information with respect to a cycle of the mechanism based on the order of priority for assignment of the analysis processing.

3. The automatic analyzer according to claim 1, wherein:
the mechanism includes an exclusive mechanism to be used for the analytical operation for each of the analysis types, and a common mechanism to be commonly used for analytical operations of the multiple analysis types;
each of the exclusive mechanisms for the analysis types has a cycle time required for the analytical operation, which differs in accordance with the analysis type; and
the evaluation value is set on the evaluation setting information to make the priority higher as the cycle time becomes longer in accordance with the analysis type.

4. The automatic analyzer according to claim 1, wherein:
the multiple analysis types include at least two types selected from a biochemical analysis, an immunity analysis, and an electrolyte analysis; and
the evaluation value is set on the evaluation setting information so that the priority differs in accordance with the analysis type.

5. The automatic analyzer according to claim 1, wherein:
the specimen type includes at least an urgent specimen and a general specimen; and
the evaluation value is set on the evaluation setting information so that the priority differs in accordance with the specimen type.

6. The automatic analyzer according to claim 1, wherein:
the multiple information elements include an information element of sameness between consecutive specimens;
a value set on the information element of sameness between consecutive specimens indicates whether a latter specimen of the consecutive specimens is the same as or different from a former specimen; and
the evaluation value is set on the evaluation setting information as a value of the sameness between consecutive specimens so that the priority given to a case of the same specimens becomes higher than the one given to a case of the different specimens.

7. The automatic analyzer according to claim 1, wherein:
the multiple information elements include at least one of a residual amount of the specimen, a time elapsing from placement of the specimen, and dilution/non-dilution of the specimen; and
the evaluation value is set on the evaluation setting information so that the priority becomes higher in the case of smaller residual amount of the specimen, dilution of the specimen, or longer time elapsing from placement of the specimen.

8. The automatic analyzer according to claim 1, wherein:
the multiple information elements include at least one of a residual amount of a reagent used for analyzing the specimen, and a time elapsing from placement of the reagent; and
the evaluation value is set on the evaluation setting information so that the priority becomes higher in the case of smaller residual amount of the reagent, or longer time elapsing from placement of the reagent.

9. The automatic analyzer according to claim 1, wherein:
the multiple information elements include at least the specimen type, the analysis type, the residual amount or the time elapsing from placement of the specimen, and the residual amount or the time elapsing from placement of the reagent used for analyzing the specimen; and
the evaluation value is set on the evaluation setting information to prioritize the information elements from the highest to the lowest in the order of the specimen type, the analysis type, the residual amount or the time elapsing from placement of the specimen, and the residual amount or the time elapsing from placement of the reagent for analyzing the specimen.

10. The automatic analyzer according to claim 1, wherein the processor provides a screen for setting the evaluation setting information based on an operation performed by the user.

11. The automatic analyzer according to claim 1, wherein:
the storage device stores multiple pieces of the evaluation setting information; and
the processor provides a screen for selecting the evaluation setting information to be applied among the multiple pieces of evaluation setting information based on an operation performed by the user.

12. The automatic analyzer according to claim 11, wherein the processor provides a screen for setting the evaluation setting information in accordance with a date and time, and a time zone.

13. The automatic analyzer according to claim 10, wherein the processor changes the priority of an item or a value of a designated information element of the evaluation setting information, or the evaluation value corresponding to the priority.

14. The automatic analyzer according to claim 10, wherein the processor switches ON/OFF with respect to application of an item of a designated information element of the evaluation setting information based on an operation of the screen by the user.
